(19) Europäisches Patentamt
European Patent Office
Office européen des brevets

(11) EP 4 129 293 A1

(12) EUROPEAN PATENT APPLICATION
published in accordance with Art. 153(4) EPC

(43) Date of publication:
**08.02.2023 Bulletin 2023/06**

(21) Application number: **21774856.5**

(22) Date of filing: **26.03.2021**

(51) International Patent Classification (IPC):
***A61K 31/53*** *(1974.07)* ***A61P 21/02*** *(2000.01)*
***A61P 25/00*** *(2000.01)* ***A61P 25/28*** *(2000.01)*
***C07D 251/22*** *(1974.07)* ***C07D 405/04*** *(1974.07)*

(52) Cooperative Patent Classification (CPC):
**A61K 31/53; A61P 21/02; A61P 25/00; A61P 25/28; C07D 251/22; C07D 405/04**

(86) International application number:
**PCT/JP2021/014251**

(87) International publication number:
**WO 2021/193982 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.03.2020 JP 2020058414**

(71) Applicant: **Kyoto University**
**Kyoto-shi, Kyoto 606-8501 (JP)**

(72) Inventors:
- **INOUE, Haruhisa**
  **Kyoto-shi, Kyoto 606-8501 (JP)**
- **IMAMURA, Keiko**
  **Kyoto-shi, Kyoto 606-8501 (JP)**
- **FURUSAWA, Makoto**
  **Fujisawa-shi, Kanagawa 251-0012 (JP)**
- **FUNATA, Masaaki**
  **Fujisawa-shi, Kanagawa 251-0012 (JP)**
- **HAYASHI, Satoru**
  **Fujisawa-shi, Kanagawa 251-0012 (JP)**
- **IMAMURA, Keisuke**
  **Fujisawa-shi, Kanagawa 251-0012 (JP)**
- **SUGIMOTO, Takahiro**
  **Fujisawa-shi, Kanagawa 251-0012 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **NERVE CELL DEGENERATION INHIBITOR**

(57) The present invention aims to provide a neuron degeneration inhibitor.

The present invention relates to a neuron degeneration inhibitor comprising a compound represented by the formula (I)

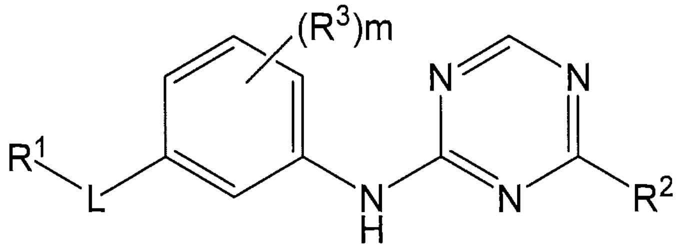

(I)

wherein each symbol is as defined in the description, or a salt thereof.

EP 4 129 293 A1

Fig. 1

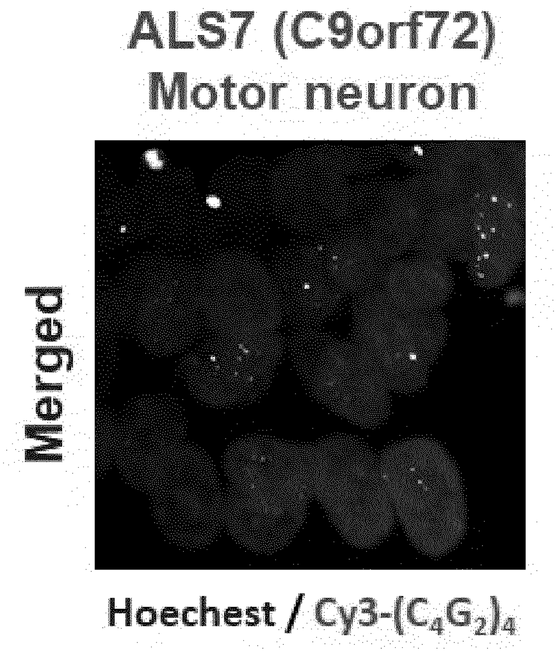

## Description

### Technical Field

The present invention relates to an agent (a medicament, a pharmaceutical composition) comprising a compound useful for the treatment of motor neuron diseases or dementia.

(Background of the Invention)

Eukaryotic genomes contain various repeat sequences, and recently, six-base repeat sequence "GGGGCC" (hereinafter also to be referred to as G4C2 repeat) located in the untranslated region of C9orf72 gene has attracted attention as a link between amyotrophic lateral sclerosis (hereinafter also to be referred to as "ALS") and frontotemporal dementia (hereinafter also to be referred to as "FTD").

ALS is a typical motor neuron disease characterized by selective degeneration of upper and lower motor neurons, with approximately 10% of patients sporadic and approximately 90% familial. So far, studies based on genetic mutations found in familial patients have been intensively conducted, and recently, abnormal expansion of the G4C2 repeat of the C9orf72 gene was identified as the most frequently observed genetic abnormality in familial and sporadic ALS patients. On the other hand, FTD is characterized by degeneration of the frontal and temporal lobes of the brain and is the second most common disease among dementia patients under the age of 65, and it was reported that the most frequently observed genetic abnormality in FTD is also abnormal expansion of the G4C2 repeat of the C9orf72 gene (Non-Patent Documents 1 and 2).

It is known that RNA transcribed from the C9orf72 gene with the abnormally expanded G4C2 repeat easily aggregates, and enfolds nuclear proteins to generate nuclear aggregates (hereinafter to be referred to as RNA foci) (Non-Patent Document 1 and 2). It is also known that the above RNA is translated into dipeptide repeat proteins (hereinafter to be referred to as DPRs) by repeat-associated non-ATG translation (hereinafter to be referred to as RAN translation), and the DPRs generate intracellular inclusions (Non-Patent Documents 3, 4 and 5). RNA foci and DPRs are considered to be the main cause of neurodegeneration in these diseases due to their cytotoxicity (Non-Patent Document 6), and effective inhibition of their generation is considered to prevent the onset of these diseases or effectively suppress the pathological progression after the onset.

So far, various model systems have been used to search for genes that can inhibit the generation of RNA foci and/or DPRs. For example, by screening using a Drosophila model in which the abnormally expanded G4C2 repeat are expressed in the compound eye, FUS (the gene responsible for ALS6) was identified as a gene whose overexpression suppresses RAN translation and compound eye neurodegeneration (Patent Document 1). In addition, an antisense oligonucleotide for the C9orf72 gene has also been developed, and has been reported to inhibit the generation of RNA foci and effectively suppress neuron death (for example, Non-Patent Document 7).

However, small molecule compounds that can effectively inhibit the generation of RNA foci and/or DPRs caused by the G4C2 repeat have not yet been reported.

### Document List

### Patent Document

Patent Document 1: JP 2018-193309

### Non-Patent Document

Non-Patent Document 1: DeJesus-Hernandez, M. et al., Neuron 72, 245-56 (2011)
Non-Patent Document 2: Renton, A.E. et al., Neuron 72, 257-68 (2011)
Non-Patent Document 3: Ash, P.E. et al., Neuron 77, 639-46 (2013)
Non-Patent Document 4: Mori, K. et al., Science 339, 1335-8 (2013)
Non-Patent Document 5: Zu, T. et al., Proc Natl Acad Sci U S A 110, E4968-77 (2013)
Non-Patent Document 6: J. Chew et al., Science, 348, 1151-1154 (2015)
Non-Patent Document 7: D. Sareen et al., Sci. Transl. Med., 5, 208ra149, doi:10.1126/scitranslmed.3007529 (2013)

## Summary of the Invention

### Problems to be Solved by the Invention

The present invention aims to find a compound capable of effectively inhibiting the generation of RNA foci and DPRs, which is considered to be the cause of neurodegeneration in motor neuron diseases and dementia represented by FTD, and to provide an agent (a medicament, a pharmaceutical composition) which can be used for the prophylaxis or treatment of the above diseases.

### Means of Solving the Problems

The present inventors have conducted intensive studies in an attempt to solve the above-mentioned problems and first found that motor neurons induced to differentiate by overexpression of Lhx3, Ngn2 and Isl1 genes from ALS patient-derived iPS cells with the abnormally expanded G4C2 repeat spontaneously generate RNA foci and DPRs, leading to neurodegeneration. The method of inducing differentiation by expressing the three genes has been developed by the present inventors as a method capable of inducing differentiation into motor neurons in a short period of time and with high synchrony (WO 2014/148646, and K. Imamura et al, Science Translational Medicine 2017, 9, eaaf3962).

Then, the present inventors have screened small molecule compounds using the above motor neurons, and found that seven compounds had an excellent inhibitory effect on the generation of RNA foci and DPRs, i.e., they could be an agent for the prophylaxis or treatment of motor neuron diseases and/or dementia caused by the repeat abnormal expansion, which resulted in the completion of the present invention.

Accordingly, the present invention provides the following.

[1] A neuron degeneration inhibitor (hereinafter also to be referred to as "the agent of the present invention") comprising a compound represented by the formula (I)

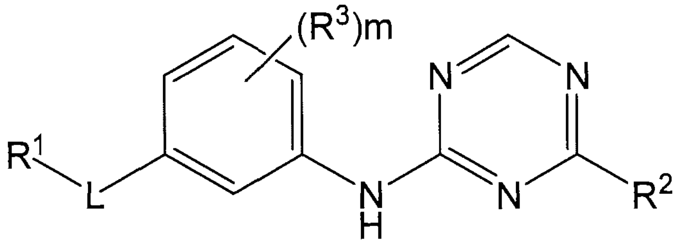

(I)

wherein

$R^1$ is a group represented by the formula (a-1) or (a-2) [0010]

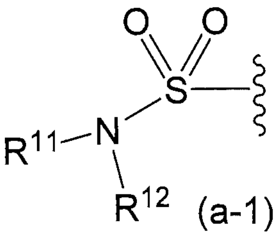

(a-1) or

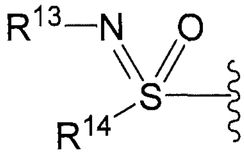

(a-2)

wherein

$R^{11}$ and $R^{12}$ are each independently a hydrogen atom or a $C_{1-6}$ alkyl group,
$R^{13}$ is a hydrogen atom, a cyano group, a $C_{1-6}$ alkyl-carbonyl group or a $C_{1-6}$ alkoxy-carbonyl group, and
$R^{14}$ is a $C_{1-6}$ alkyl group, a $C_{3-8}$ cycloalkyl group or a $C_{6-14}$ aryl group,

$R^2$ is a group represented by the formula (b-1)-(b-3) [0012]

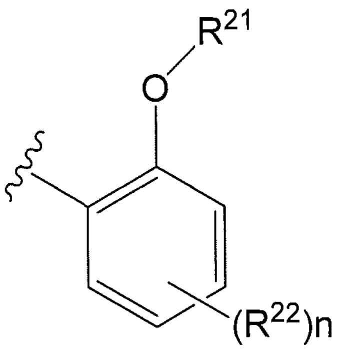
(b-1)

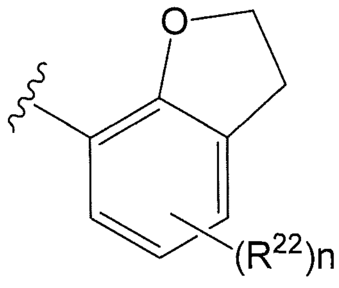
(b-2)

or

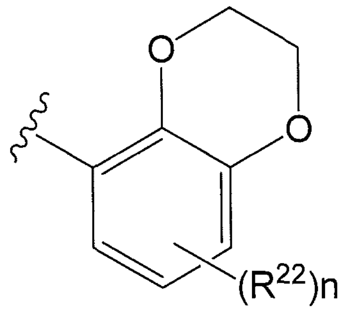
(b-3)

wherein

> $R^{21}$ is a $C_{1-6}$ alkyl group, a $C_{6-14}$ aryl group optionally substituted by halogen atom(s), or a $C_{7-16}$ aralkyl group optionally substituted by halogen atom(s),
> $R^{22}$ is each independently a halogen atom, a cyano group, a $C_{1-6}$ alkyl group or a $C_{1-6}$ alkoxy group, and
> n is 0, 1 or 2,

$R^3$ is each independently a halogen atom, a cyano group, a $C_{1-6}$ alkyl group or a $C_{1-6}$ alkoxy group,
m is 0, 1 or 2, and
L is a $C_{1-3}$ alkylene group,
or a salt thereof.

[2] The neuron degeneration inhibitor according to the above-mentioned [1], wherein

$R^{11}$ and $R^{12}$ are both hydrogen atoms,
$R^{13}$ is a hydrogen atom or a $C_{1-6}$ alkoxy-carbonyl group,
$R^{14}$ is a $C_{1-6}$ alkyl group,
$R^{21}$ is a $C_{1-6}$ alkyl group, or a $C_{7-16}$ aralkyl group optionally substituted by halogen atom(s),
$R^{22}$ is a halogen atom,
n is 0 or 1,
m is 0, and
L is a methylene group.

[3] The neuron degeneration inhibitor according to the above-mentioned [1], wherein the compound represented by the formula (I) or a salt thereof is selected from

(1) 4-(4-fluoro-2-methoxyphenyl)-N-{3-[(S-methanesulfonimidoyl)methyl]phenyl}-1,3,5-triazin-2-amine,
(2) 1-(3-{[4-(2-methoxyphenyl)-1,3,5-triazin-2-yl]amino}phenyl)methanesulfonamide,
(3) 1-(3-{[4-(4-chloro-2-methoxyphenyl)-1,3,5-triazin-2-yl]amino}phenyl)methanesulfonamide,
(4) 1-[3-((4-[2-(benzyloxy)phenyl]-1,3,5-triazin-2-yl}amino)phenyl]methanesulfonamide,
(5) 4-{2-[(3,4-dichlorophenyl)methoxy]phenyl}-N-{3-[(S-methanesulfonimidoyl)methyl]phenyl}-1,3,5-triazin-2-amine,
(6) ethyl {[(3-{[4-(2,3-dihydro-1,4-benzodioxin-5-yl)-1,3,5-triazin-2-yl]amino}phenyl)methyl] (methyl)oxo-$\lambda^6$-sulfanylidene}carbamate,
(7) ethyl {[(3-{[4-(2,3-dihydro-1-benzofuran-7-yl)-1,3,5-triazin-2-yl]amino}phenyl)methyl] (methyl)oxo-$\lambda^6$-sulfanylidene}carbamate,

and salts thereof.
[4] The neuron degeneration inhibitor according to any of the above-mentioned [1]-[3], which is used as an agent for the prophylaxis or treatment of a motor neuron disease or dementia.
[5] The neuron degeneration inhibitor according to the above-mentioned [4], wherein the motor neuron disease or dementia is a motor neuron disease or dementia involving abnormal expansion of hexanucleotide repeat.
[6] The neuron degeneration inhibitor according to the above-mentioned [4] or [5], wherein the motor neuron disease

is amyotrophic lateral sclerosis.

[7] The neuron degeneration inhibitor according to the above-mentioned [4] or [5], wherein the dementia is frontotemporal dementia.

[8] A method for inhibiting neuron degeneration in a mammal, which comprises administering an effective amount of a compound or salt as defined in any of the above-mentioned [1]-[3] to the mammal.

[9] A method for preventing or treating a neuron degeneration disease in a mammal, which comprises administering an effective amount of a compound or salt as defined in any of the above-mentioned [1]-[3] to the mammal.

[10] A compound or salt as defined in any of the above-mentioned [1]-[3] for use in the prophylaxis or treatment of a neuron degeneration disease.

[11] Use of a compound or salt as defined in any of the above-mentioned [1]-[3] for the manufacture of an agent for the prophylaxis or treatment of a neuron degeneration disease.

**Effect of the Invention**

According to the present invention, an agent which has an excellent inhibitory effect on the generation of RNA foci and DPRs in neurodegenerative diseases involving abnormal expansion of the G4C2 repeat of the C9orf72 gene, and can be used for the prophylaxis or treatment of the above diseases can be provided.

**Brief Description of the Drawings**

[Figure 1]
Figure 1 is a fluorescence microscopy image of RNA foci visualized using a fluorescent probe in motor neurons differentiated from ALS7 (C9orf72) cells.

(Detailed Description of the Invention)

The present invention is explained in detail in the following.

The present invention provides a neuron degeneration inhibitor comprising a compound represented by the following formula (I) or a salt thereof (hereinafter also to be referred to as "compound (I)") as an active ingredient.

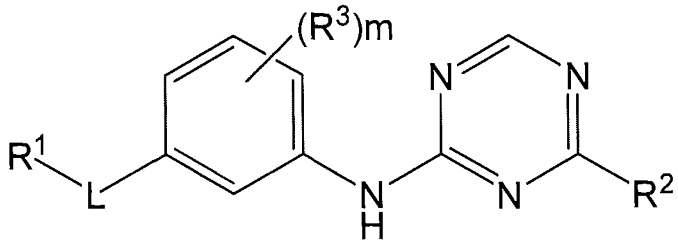

(I)

wherein

$R^1$ is a group represented by the formula (a-1) or (a-2) [0022]

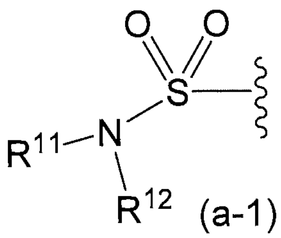

(a-1) or

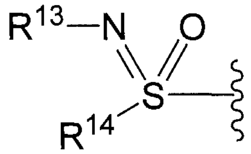

(a-2)

wherein

$R^{11}$ and $R^{12}$ are each independently a hydrogen atom or a $C_{1-6}$ alkyl group,
$R^{13}$ is a hydrogen atom, a cyano group, a $C_{1-6}$ alkyl-carbonyl group or a $C_{1-6}$ alkoxy-carbonyl group, and
$R^{14}$ is a $C_{1-6}$ alkyl group, a $C_{3-8}$ cycloalkyl group or a $C_{6-14}$ aryl group,

$R^2$ is a group represented by the formula (b-1)-(b-3)

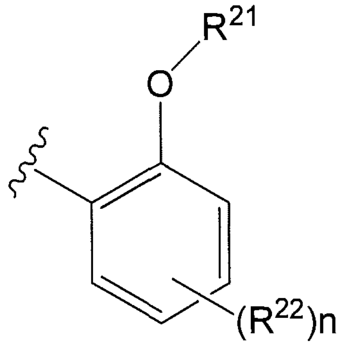

(b-1)

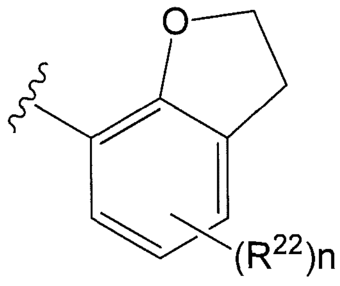

(b-2) or

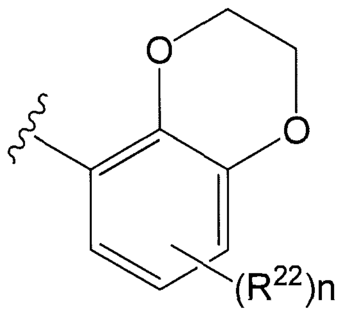

(b-3)

wherein

$R^{21}$ is a $C_{1-6}$ alkyl group, a $C_{6-14}$ aryl group optionally substituted by halogen atom(s), or a $C_{7-16}$ aralkyl group optionally substituted by halogen atom(s),
$R^{22}$ is each independently a halogen atom, a cyano group, a $C_{1-6}$ alkyl group or a $C_{1-6}$ alkoxy group, and
n is 0, 1 or 2,

$R^3$ is each independently a halogen atom, a cyano group, a $C_{1-6}$ alkyl group or a $C_{1-6}$ alkoxy group,
m is 0, 1 or 2, and
L is a $C_{1-3}$ alkylene group.

The definition of each substituent used as used herein, is explained in detail in the following. Unless otherwise specified, each substituent has the following definition.

As used herein, examples of the "halogen atom" include fluorine, chlorine, bromine and iodine.

As used herein, examples of the "$C_{1-6}$ alkyl group" include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl and 2-ethylbutyl.

As used herein, examples of the "$C_{3-8}$ cycloalkyl group" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl and adamantyl.

As used herein, examples of the "$C_{6-14}$ aryl group" include phenyl, 1-naphthyl, 2-naphthyl, 1-anthryl, 2-anthryl and 9-anthryl.

As used herein, examples of the "$C_{7-16}$ aralkyl group" include benzyl, phenethyl, naphthyl methyl and phenylpropyl.

As used herein, examples of the "$C_{1-6}$ alkoxy group" include methoxy, ethoxy, propoxy, isopropoxy, butoxy, isobutoxy, sec-butoxy, tert-butoxy, pentyloxy and hexyloxy.

As used herein, examples of the "$C_{1-6}$ alkyl-carbonyl group" include acetyl, propanoyl, butanoyl, 2-methylpropanoyl, pentanoyl, 3-methylbutanoyl, 2-methylbutanoyl, 2,2-dimethylpropanoyl, hexanoyl and heptanoyl.

As used herein, examples of the "$C_{1-6}$ alkoxy-carbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl and hexyloxycarbonyl.

As used herein, examples of the "$C_{1-3}$ alkylene group" include $-CH_2-$, $-(CH_2)_2-$, $-(CH_2)_3-$, $-CH(CH_3)-$, $-CH_2CH(CH_3)-$, $-CH(CH_3)CH_2-$, $-C(CH_3)_2-$ and $-CH(C_2H_5)-$.

The definition of each symbol in the formula (I) is explained in detail in the following.

$R^1$ is a group represented by the formula (a-1) or (a-2)

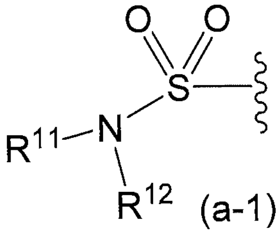

(a-1) or

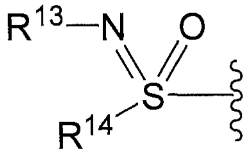

(a-2) .

$R^{11}$ and $R^{12}$ are each independently a hydrogen atom or a $C_{1-6}$ alkyl group, $R^{13}$ is a hydrogen atom, a cyano group, a $C_{1-6}$ alkyl-carbonyl group or a $C_{1-6}$ alkoxy-carbonyl group, and $R^{14}$ is a $C_{1-6}$ alkyl group, a $C_{3-8}$ cycloalkyl group or a $C_{6-14}$ aryl group.

$R^{11}$ and $R^{12}$ are preferably both hydrogen atoms.

$R^{13}$ is preferably a hydrogen atom or a $C_{1-6}$ alkoxy-carbonyl group (e.g., ethoxycarbonyl), particularly preferably a hydrogen atom.

$R^{14}$ is preferably a $C_{1-6}$ alkyl group (e.g., methyl), particularly preferably a methyl group.

$R^1$ is preferably a group represented by the formula (a-2) .

$R^2$ is a group represented by the formula (b-1)-(b-3)

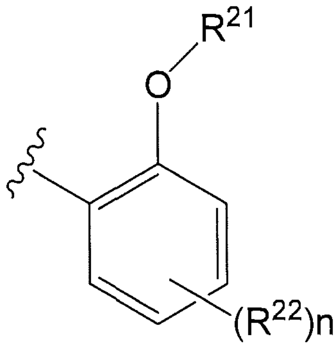

(b-1)

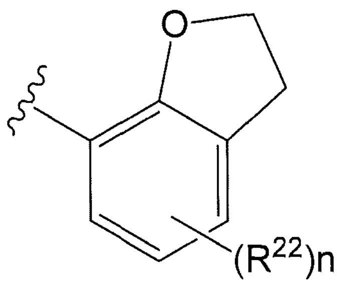

(b-2) or

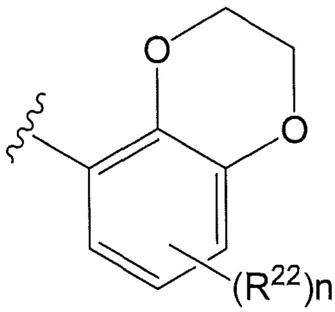

(b-3) .

$R^{21}$ is a $C_{1-6}$ alkyl group, a $C_{6-14}$ aryl group optionally substituted by halogen atom(s), or a $C_{7-16}$ aralkyl group optionally substituted by halogen atom(s), $R^{22}$ is each independently a halogen atom, a cyano group, a $C_{1-6}$ alkyl group or a $C_{1-6}$ alkoxy group, and n is 0, 1 or 2.

$R^{21}$ is preferably a $C_{1-6}$ alkyl group (e.g., methyl), or a $C_{7-16}$ aralkyl group (e.g., benzyl) optionally substituted by halogen atom(s) (e.g., a chlorine atom), more preferably a $C_{1-6}$ alkyl group (e.g., methyl), particularly preferably a methyl group.

$R^{22}$ is preferably a halogen atom (e.g., a fluorine atom, a chlorine atom), particularly preferably a fluorine atom.

n is preferably 0 or 1, particularly preferably 1.

$R^2$ is preferably a group represented by the formula (b-1) .

$R^3$ is each independently a halogen atom, a cyano group, a $C_{1-6}$ alkyl group or a $C_{1-6}$ alkoxy group.

m is 0, 1 or 2.

m is preferably 0.

L is a $C_{1-3}$ alkylene group.

L is preferably a methylene group.

Preferable embodiment of compound (I) includes the following compounds.

Compound (I) wherein

$R^1$ is a group represented by the formula (a-1) or (a-2),
$R^{11}$ and $R^{12}$ are both hydrogen atoms,
$R^{13}$ is a hydrogen atom or a $C_{1-6}$ alkoxy-carbonyl group (e.g., ethoxycarbonyl),
$R^{14}$ is a $C_{1-6}$ alkyl group (e.g., methyl),
$R^2$ is a group represented by the formula (b-1)-(b-3),
$R^{21}$ is a $C_{1-6}$ alkyl group (e.g., methyl), or a $C_{7-16}$ aralkyl group (e.g., benzyl) optionally substituted by halogen atom(s) (e.g., a chlorine atom),

$R^{22}$ is a halogen atom (e.g., a fluorine atom, a chlorine atom), n is 0 or 1,
m is 0, and
L is a methylene group.

Specific examples of compound (I) include the following compounds.

(1) 4-(4-fluoro-2-methoxyphenyl)-N-{3-[(S-methanesulfonimidoyl)methyl]phenyl}-1,3,5-triazin-2-amine (Compound No.1),
(2) 1-(3-{[4-(2-methoxyphenyl)-1,3,5-triazin-2-yl]amino}phenyl)methanesulfonamide (Compound No.2),
(3) 1-(3-{[4-(4-chloro-2-methoxyphenyl)-1,3,5-triazin-2-yl]amino}phenyl)methanesulfonamide (Compound No.3),
(4) 1-[3-({4-[2-(benzyloxy)phenyl]-1,3,5-triazin-2-yl}amino)phenyl]methanesulfonamide (Compound Nos.4a, 4b),
(5) 4-{2-[(3,4-dichlorophenyl)methoxy]phenyl}-N-{3-[(S-methanesulfonimidoyl)methyl]phenyl}-1,3,5-triazin-2-amine (Compound No.5),
(6) ethyl {[(3-{[4-(2,3-dihydro-1,4-benzodioxin-5-yl)-1,3,5-triazin-2-yl]amino}phenyl)methyl] (methyl)oxo-$\lambda^6$-sulfanylidene}carbamate (Compound No.6),
(7) ethyl {[(3-{[4-(2,3-dihydro-1-benzofuran-7-yl)-1,3,5-triazin-2-yl]amino}phenyl)methyl] (methyl)oxo-$\lambda^6$-sulfanylidene}carbamate (Compound No.7),

and salts thereof.

When compound (I) is a salt, it is preferably a pharmacologically acceptable salt. Examples of such salt include salts with inorganic base, salts with organic base, salts with inorganic acid, salts with organic acid, salts with basic amino acid, and salts with acidic amino acid.

Preferable examples of the salt with inorganic base include alkali metal salts such as sodium salt, potassium salt and the like; alkaline-earth metal salts such as calcium salt, magnesium salt and the like; aluminium salt; and ammonium salt.

Preferable examples of the salt with organic base include salts with trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine and N,N-dibenzyl ethylene diamine.

Preferable examples of the salt with inorganic acid include salts with hydrochloric acid, hydrobromic acid, nitric acid, sulfuric acid and phosphoric acid.

Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid and p-toluenesulfonic acid.

Preferable examples of the salt with basic amino acid include salts with arginine, lysine and ornithine.

Preferable examples of the salt with acidic amino acid include salts with aspartic acid and glutamic acid.

When compound (I) is obtained as a free form, it can be converted to the objective salt according to a method known per se. When compound (I) is obtained as a salt, it can be converted to the objective free form or the other salt according to a method known per se.

The term "neuron degeneration" as used herein refers to the occurrence of any one or more abnormalities of neurite atrophy, neurite fragmentation, neurite disappearance, cell body atrophy, cell body fragmentation and cell body disappearance. In general, neuron degeneration can be detected and evaluated using cell death (practically, as the inverse of the number of surviving neurons) as an index.

Moreover, for neurodegeneration caused by abnormal expansion of repeat sequence with hexa- (or tri-) nucleotide unit, neurodegeneration can be detected and evaluated using RNA foci consisting of RNA transcribed from the repeat sequence, or DPRs generated by RAN translation from the RNA, as an index. Both RNA foci and DPRs can be detected and quantified by well-known conventional methods.

For RNA foci, for example, the neurons are analyzed by FISH (fluorescence in situ hybridization) method using an oligonucleotide (preferably labeled with fluorescence etc.) containing sequence complementary to the repeat sequence as a probe, and the RNA foci may be detected as dots of the fluorescent signal present in the nuclei of the neurons (for example, dots having a diameter of 0.60 $\mu$m or more). The RNA foci generation level may be quantitatively evaluated by measuring the number of dots per neuron or per unit area (for example, the observation field area, the nuclear staining area in the observation field, etc.).

For example, the DPRs may be detected and quantified by analyzing neuron-derived lysates or extracts by well-known and conventional immunological techniques (for example, ELISA method) using antibodies against DPRs that may be generated from the hexa- (or tri-) nucleotide repeat sequences. Alternatively, by immunostaining of the neurons using the above antibodies, the DPRs may be detected and quantified as the antibody-positive dots.

In one embodiment, using motor neurons derived from iPS cells of ALS patients, the cell degeneration inhibitory rate of motor neurons by a certain compound (test compound) may be calculated using the following formula.

$$\text{Motor neuron degeneration inhibitory activity of test compound} = ((X-C)/(T-C)) \times 100$$

X: Number of motor neurons in the test compound group y days after the start of the culture,
C: Number of motor neurons in the DMSO group y days after the start of the culture,
T: Number of motor neurons x days after the start of the culture

Here, x is selected from any day before spontaneous cell death occurs in the subject, and y is selected from any day during spontaneous cell death occurs in the subject.

Since compound (I) has an excellent inhibitory effect on the generation of RNA foci and DPRs caused by abnormal expansion of the G4C2 repeat, the agent of the present invention can be suitably used as agents for the prophylaxis or treatment of neurodegenerative diseases involving abnormal expansion of the repeat. Moreover, since it is considered that there is a common mechanism that is sequence-independent in the process of the generation of RNA foci and DPRs from hexa- (or tri-) nucleotide repeat, the agent of the present invention is expected as agents for the prophylaxis or treatment of all neurodegenerative diseases involving abnormal expansion of hexa- (or tri-) nucleotide. The neurodegenerative diseases include motor neuron diseases and dementia.

Examples of the motor neuron disease include amyotrophic lateral sclerosis (ALS), progressive bulbar paralysis, progressive muscular atrophy, primary lateral sclerosis, progressive pseudobulbar paralysis, spinal muscular atrophy, Parkinson's disease, multiple-system atrophy, Huntington's disease, spinocerebellar degeneration, myotonic dystrophy, fragile X syndrome-associated diseases, oculopharyngeal myopathy, Fuchs corneal dystrophy, spherotomy muscular atrophy and the like. As used herein, these diseases are sometimes referred to as "motor neuron diseases". Among these, amyotrophic lateral sclerosis, spherotomy muscular atrophy, myotonic dystrophy, Parkinson's disease, Huntington's disease, fragile X syndrome-associated diseases, and spinal muscular atrophy are particularly preferably exemplified as target motor neuron diseases for the agent according to the present invention.

Examples of the dementia include frontotemporal dementia (FTD), Lewy body dementia and the like, and FTD is a particularly suitable target dementia disease.

The agent for the prophylaxis or treatment can be used for the above diseases, regardless of whether they are sporadic or familial.

The agent for the prophylaxis or treatment can be used as agents for the prophylaxis or treatment of the above diseases in mammals (e.g., mice, rats, hamster, rabbits, cats, dogs, cows, sheep, monkeys, humans and the like).

The agent of the present invention may contain one kind of compound (I), or may contain two or more kinds thereof in combination.

Compound (I) is superior in vivo kinetics (e.g., plasma drug half-life, intracerebral transferability, metabolic stability), shows low toxicity (e.g., more superior as a medicament in terms of acute toxicity, chronic toxicity, genetic toxicity, reproductive toxicity, cardiotoxicity, drug interaction, carcinogenicity etc.). Compound (I) is directly used as a medicament or a pharmaceutical composition mixed with a pharmaceutically acceptable carrier or the like to be orally or parenterally administered to mammals (e.g., humans, monkeys, cows, horses, pigs, mice, rats, hamsters, rabbits, cats, dogs, sheep and goats) in safety. Examples of the "parenteral" include intravenous, intramuscular, subcutaneous, intra-organ, intranasal, intradermal, instillation, intracerebral, intrarectal, intravaginal, intraperitoneal and intratumor administrations, administration to the vicinity of tumor etc. and direct administration to the lesion.

While the dose of compound (I) varies depending on the administration route, symptom and the like, when, for example, compound (I) is orally administered to a patient with amyotrophic lateral sclerosis (adult, body weight 40 - 80 kg, for example, 60 kg), it is, for example, 0.001 - 1000 mg/kg body weight/day, preferably 0.01 - 100 mg/kg body weight/day, more preferably 0.1 - 10 mg/kg body weight/day. This amount can be administered in 1 to 3 portions per day.

The agent of the present invention can use compound (I) alone or as a pharmaceutical composition containing compound (I) and a pharmaceutically acceptable carrier according to a method known per se as a production method of a pharmaceutical preparation (e.g., the method described in the Japanese Pharmacopoeia etc.). The agent (medicament, pharmaceutical composition) of the present invention can be safely administered in the form of, for example, tablet (including sugar-coated tablet, film-coated tablet, sublingual tablet, orally disintegrating tablet, buccal and the like), pill, powder, granule, capsule (including soft capsule, microcapsule), troche, syrup, liquid, emulsion, suspension, release control preparation (e.g., immediate-release preparation, sustained-release preparation, sustained-release microcapsule), aerosol, film (e.g., orally disintegrating film, oral mucosa-adhesive film), injection (e.g., subcutaneous injection, intravenous injection, intramuscular injection, intraperitoneal injection), drip infusion, transdermal absorption type preparation, ointment, lotion, adhesive preparation, suppository (e.g., rectal suppository, vaginal suppository), pellet, nasal preparation, pulmonary preparation (inhalant), eye drop and the like, orally or parenterally (e.g., intravenous, intramuscular, subcutaneous, intraorgan, intranasal, intradermal, instillation, intracerebral, intrarectal, intravaginal, intraperitoneal administrations, and administration to the lesion).

As the aforementioned "pharmaceutically acceptable carrier", various organic or inorganic carriers conventionally used as preparation materials (starting materials) can be used. For example, excipient, lubricant, binder, disintegrant and the like are used for solid preparations, and solvent, solubilizing agent, suspending agent, isotonicity agent, buffer, soothing agent and the like are used for liquid preparations. Where necessary, preparation additives such as preservative, antioxidant, colorant, sweetening agent and the like can also be used.

Examples of the excipient include lactose, sucrose, D-mannitol, starch, corn starch, crystalline cellulose and light anhydrous silicic acid.

Examples of the lubricant include magnesium stearate, calcium stearate, talc and colloidal silica.

Examples of the binder include crystalline cellulose, sucrose, D-mannitol, dextrin, hydroxypropylcellulose, hydroxypropylmethylcellulose, polyvinylpyrrolidone, starch, sucrose, gelatin, methylcellulose and carboxymethylcellulose sodium.

Examples of the disintegrant include starch, carboxymethylcellulose, carboxymethylcellulose calcium, sodium carboxymethyl starch and L-hydroxypropylcellulose.

Examples of the solvent include water for injection, alcohol, propylene glycol, Macrogol, sesame oil, corn oil and olive oil.

Examples of the solubilizing agent include polyethylene glycol, propylene glycol, D-mannitol, benzyl benzoate, ethanol, trisaminomethane, cholesterol, triethanolamine, sodium carbonate and sodium citrate.

Examples of the suspending agent include surfactants such as stearyl triethanolamine, sodium lauryl sulfate, laurylaminopropionic acid, lecithin, benzalkonium chloride, benzetonium chloride, glycerin monostearate and the like; and hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like.

Examples of the isotonicity agent include glucose, D-sorbitol, sodium chloride, glycerin and D-mannitol.

Examples of the buffer include buffer solutions such as phosphates, acetates, carbonates and citrates.

Examples of the soothing agent include benzyl alcohol.

Examples of the preservative include p-oxybenzoates, chlorobutanol, benzyl alcohol, phenylethyl alcohol, dehydroacetic acid and sorbic acid.

Examples of the antioxidant include sulfite, ascorbic acid and $\alpha$-tocopherol.

While the pharmaceutical composition (formulation) varies according to the dosage form, administration method, carrier and the like, it can be produced according to a conventional method by adding compound (I) in a proportion of generally 0.01 - 100%(w/w), preferably 0.1 - 95%(w/w), of the total amount of the preparation.

Compound (I) may be used alone or in combination of two or more thereof, as an agent (a medicine, a pharmaceutical composition). As used herein, unless otherwise specified, compound (I) also includes a combination of a plurality thereof.

Moreover, compound (I) can be used in combination with other active ingredients (hereinafter to be abbreviated as concomitant drug).

Examples of the concomitant drug include the following. benzodiazepine (chlordiazepoxide, diazepam, potassium clorazepate, lorazepam, clonazepam, alprazolam etc.), L-type calcium channel inhibitor (pregabalin etc.), tricyclic or tetracyclic antidepressant (imipramine hydrochloride, amitriptyline hydrochloride, desipramine hydrochloride, clomipramine hydrochloride etc.), selective serotonin reuptake inhibitor (fluvoxamine maleate, fluoxetine hydrochloride, citalopram hydrobromide, sertraline hydrochloride, paroxetine hydrochloride, escitalopram oxalate etc.), serotonin-noradrenaline reuptake inhibitor (venlafaxine hydrochloride, duloxetine hydrochloride, desvenlafaxine hydrochloride etc.), noradrenaline reuptake inhibitor (reboxetine mesylate etc.), noradrenaline-dopamine reuptake inhibitor (bupropion hydrochloride etc.), mirtazapine, trazodone hydrochloride, nefazodone hydrochloride, bupropion hydrochloride, setiptiline maleate, 5-$HT_{1A}$ agonist (buspirone hydrochloride, tandospirone citrate, osemozotan hydrochloride etc.), 5-$HT_3$ antagonist (Cyamemazine etc.), heart non-selective $\beta$ inhibitor (propranolol hydrochloride, oxprenolol hydrochloride etc.), histamine $H_1$ antagonist (hydroxyzine hydrochloride etc.), therapeutic drug for schizophrenia (chlorpromazine, haloperidol, sulpiride, clozapine, trifluoperazine hydrochloride, fluphenazine hydrochloride, olanzapine, quetiapine fumarate, risperidone, aripiprazole etc.), CRF antagonist, other antianxiety drug (meprobamate etc.), tachykinin antagonist (MK-869, saredutant etc.), medicament that acts on metabotropic glutamate receptor, CCK antagonist, $\beta$3 adrenaline antagonist (amibegron hydrochloride etc.), GAT-1 inhibitor (tiagabine hydrochloride etc.), N-type calcium channel inhibitor, carbonic anhydrase II inhibitor, NMDA glycine moiety agonist, NMDA antagonist (memantine etc.), peripheral benzodiazepine receptor agonist, vasopressin antagonist, vasopressin V1b antagonist, vasopressin V1a antagonist, phosphodiesterase inhibitor, opioid antagonist, opioid agonist, uridine, nicotinic acid receptor agonist, thyroid hormone (T3, T4), TSH, TRH, MAO inhibitor (phenelzine sulfate, tranylcypromine sulfate, moclobemide etc.), 5-$HT_{2A}$ antagonist, 5-$HT_{2A}$ inverse agonist, COMT inhibitor (entacapone etc.), therapeutic drug for bipolar disorder (lithium carbonate, sodium valproate, lamotrigine, riluzole, felbamate etc.), cannabinoid CB1 antagonist (rimonabant etc.), FAAH inhibitor, sodium channel inhibitor, anti-ADHD drug (methylphenidate hydrochloride, methamphetamine hydrochloride etc.), therapeutic drug for alcoholism, therapeutic drug for autism, therapeutic drug for chronic fatigue syndrome, therapeutic drug for

spasm, therapeutic drug for fibromyalgia syndrome, therapeutic drug for headache, therapeutic drug for insomnia (etizolam, zopiclone, triazolam, zolpidem, ramelteon, indiplon etc.), therapeutic drug for quitting smoking, therapeutic drug for myasthenia gravis, therapeutic drug for cerebral infarction, therapeutic drug for mania, therapeutic drug for hypersomnia, therapeutic drug for pain, therapeutic drug for dysthymia, therapeutic drug for autonomic ataxia, therapeutic drug for male and female sexual dysfunction, therapeutic drug for migraine, therapeutic drug for pathological gambler, therapeutic drug for restless legs syndrome, therapeutic drug for substance addiction, therapeutic drug for alcohol-related syndrome, therapeutic drug for irritable bowel syndrome, therapeutic drug for Alzheimer's disease (donepezil, galanthamine, memantine, rivastigmine etc.), therapeutic drug for Parkinson's disease (levodopa, carbidopa, benserazide, selegiline, zonisamide, entacapone, amantadine, talipexole, pramipexole, apomorphine, cabergoline, bromocriptine, istradefylline, trihexyphenidyl, promethazine, pergolide, etc.), therapeutic drug for Huntington's disease (chlorpromazine hydrochloride, haloperidol, reserpine etc.), therapeutic drug for Gaucher's disease (imiglucerase, taliglucerase alfa, velaglucerase alfa, eliglustat, miglustat, etc.), therapeutic drug for ALS (riluzole etc., neurotrophic factor etc.), therapeutic drug for multiple sclerosis (molecular target drug such as fingolimod, interferon beta 1b, natalizumab and the like, etc.), antiepilepsy drug (phenytoin, carbamazepine, phenobarbital, primidone, zonisamide, sodium valproate, ethosuximide, diazepam, nitrazepam, clonazepam, clobazam, gabapentin, topiramate, lamotrigine, levetiracetam, stiripentol, rufinamide, etc.), therapeutic drug for lipid abnormality such as cholesterol-lowering drug (statin series (pravastatin sodium, atorvastatin, simvastatin, rosuvastatin etc.), fibrate (clofibrate etc.), squalene synthetase inhibitor), therapeutic drug for abnormal behavior or suppressant of dromomania due to dementia (sedatives, antianxiety drug etc.), apoptosis inhibitor, antiobesity drug, therapeutic drug for diabetes, therapeutic drug for hypertension, therapeutic drug for hypotension, therapeutic drug for rheumatism (DMARD), anti-cancer agent, therapeutic drug for parathyroid (PTH), calcium receptor antagonist, sex hormone or a derivative thereof (progesterone, estradiol, estradiol benzoate etc.), neuronal differentiation promoter, nerve regeneration promoter, non-steroidal anti-inflammatory drug (meloxicam, tenoxicam, indomethacin, ibuprofen, celecoxib, rofecoxib, aspirin etc.), steroid (dexamethasone, cortisone acetate etc.), anti-cytokine drug (TNF inhibitor, MAP kinase inhibitor etc.), antibody medicament, nucleic acid or nucleic acid derivative, aptamer drug and the like.

By combining compound (I) and a concomitant drug, a superior effect such as

(1) the dose can be reduced as compared to single administration of compound (I) or a concomitant drug,
(2) the drug to be combined with compound (I) can be selected according to the condition of patients (mild case, severe case and the like),
(3) the period of treatment can be set longer by selecting a concomitant drug having different action and mechanism from compound (I),
(4) a sustained treatment effect can be designed by selecting a concomitant drug having different action and mechanism from compound (I),
(5) a synergistic effect can be afforded by a combined use of compound (I) and a concomitant drug, and the like, can be achieved.

Hereinafter compound (I) and a concomitant drug used in combination are referred to as the "combination agent of the present invention".

When using the combination agent of the present invention, the administration time of compound (I) and the concomitant drug is not restricted, and compound (I) or a pharmaceutical composition thereof and the concomitant drug or a pharmaceutical composition thereof can be administered to an administration subject simultaneously, or may be administered at different times. The dosage of the concomitant drug may be determined according to the dose clinically used, and can be appropriately selected depending on an administration subject, administration route, disease, combination and the like.

The administration mode of the concomitant drug of the present invention is not particularly restricted, and it is sufficient that compound (I) and the concomitant drug are combined in administration. Examples of such administration mode include the following methods:
(1) administration of a single preparation obtained by simultaneously processing compound (I) and the concomitant drug, (2) simultaneous administration of two kinds of preparations of compound (I) and the concomitant drug, which have been separately produced, by the same administration route, (3) administration of two kinds of preparations of compound (I) and the concomitant drug, which have been separately produced, by the same administration route in a staggered manner, (4) simultaneous administration of two kinds of preparations of compound (I) and the concomitant drug, which have been separately produced, by different administration routes, (5) administration of two kinds of preparations of compound (I) and the concomitant drug, which have been separately produced, by different administration routes in a staggered manner (for example, administration in the order of compound (I) and the concomitant drug, or in the reverse order) and the like.

The combination agent of the present invention exhibits low toxicity. For example, compound (I) or(and) the

aforementioned concomitant drug can be combined with a pharmacologically acceptable carrier according to the known method to prepare a pharmaceutical composition such as tablets (including sugar-coated tablet and film-coated tablet), powders, granules, capsules (including soft capsule), liquids, injections, suppositories and sustained-release agents. These compositions can be administered safely orally or non-orally (e.g., topical, rectal, intravenous administration). Injection can be administered intravenously, intramuscularly, subcutaneously, or by intraorgan administration or directly to the lesion.

Examples of the pharmacologically acceptable carriers usable for the production of the combination agent of the present invention include those similar to the above-mentioned carriers.

The mixing ratio of compound (I) to the concomitant drug in the combination agent of the present invention can be appropriately selected depending on an administration subject, administration route, diseases and the like.

For example, the content of compound (I) in the combination agent of the present invention differs depending on the form of a preparation, and usually from about 0.01 to about 100 wt%, preferably from about 0.1 to about 50 wt%, further preferably from about 0.5 to about 20 wt%, based on the preparation.

The content of the concomitant drug in the combination agent of the present invention differs depending on the form of a preparation, and usually from about 0.01 to about 100 wt%, preferably from about 0.1 to about 50 wt%, further preferably from about 0.5 to about 20 wt%, based on the preparation.

## Examples

The present invention is explained in detail in the following by referring to Examples and Experimental Examples, which are not to be construed as limitative, and the invention may be changed within the scope of the present invention.

The test compounds used in the following Experimental Example are as follows.

Table 1

| Compound No. | Structure Formula | Compound Name |
|---|---|---|
| 1 | 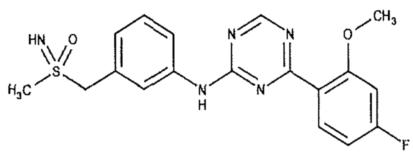 | 4-(4-fluoro-2-methoxyphenyl)-N-{3-[(S-methanesulfonimidoyl)methyl ]phenyl}-1,3,5-triazin-2-amine |
| 2 | 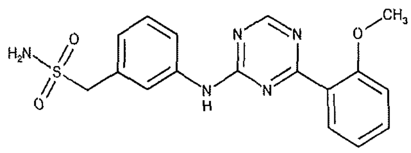 | 1-(3-{ [4-(2-methoxyphenyl)-1,3,5-triazin-2-yl]amino} phenyl)methanesulf onamide |
| 3 | 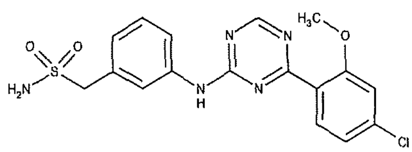 | 1-(3-{[4-(4-chloro-2-methoxyphenyl)-1,3,5-triazin-2-yl]amino}phenyl)methanesulf onamide |
| 4a | 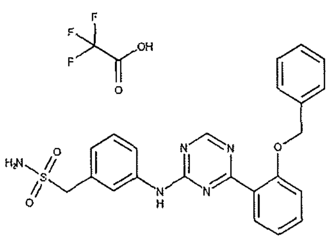 | 1-[3-({4-[2-(benzyloxy)phenyl]-1,3,5-triazin-2-yl} amino)phenyl]methanesulf onamide trifluoroacetate |

(continued)

| Compound No. | Structure Formula | Compound Name |
|---|---|---|
| 4b | 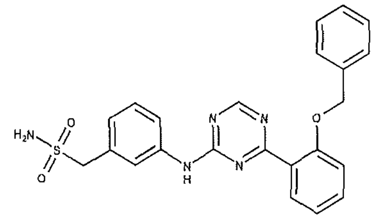 | 1-[3-({4-[2-(benzyloxy)phenyl]-1,3,5-triazin-2-yl} amino)phenyl]methanesulf onamide |
| 5 | 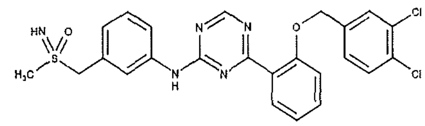 | 4-{2-[(3,4-dichlorophenyl)methoxy]phen yl}-N-{3-[(S-methanesulfonimidoyl)methyl ]phenyl}-1,3,5-triazin-2-amine |
| 6 | 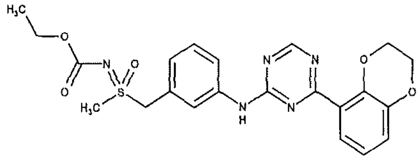 | ethyl {[(3-{[4-(2,3-dihydro-1,4-benzodioxin-5-yl)-1,3,5-triazin-2-yl]amino}phenyl)methyl] (met hyl)oxo-$\lambda^6$-sulfanylidene}carbamate |
| 7 | 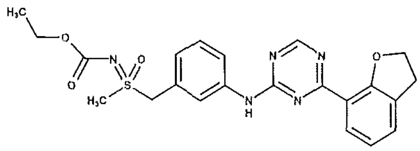 | ethyl {[(3-{[4-(2,3-dihydro-1-benzofuran-7-yl)-1,3,5-triazin-2-yl]amino}phenyl)methyl] (met hyl)oxo-$\lambda^6$-sulfanylidene}carbamate |

4-(4-Fluoro-2-methoxyphenyl)-N-{3-[(S-methanesulfonimidoyl)methyl]phenyl}-1,3,5-triazin-2-amine (Compound No.1) is described in Example 2 of WO 2012/160034.

1-(3-{[4-(2-Methoxyphenyl)-1,3,5-triazin-2-yl]amino}phenyl)methanesulfonamide (Compound No.2) is described as Compound B1 in WO 2011/116951.

1-(3-{[4-(4-Chloro-2-methoxyphenyl)-1,3,5-triazin-2-yl]amino}phenyl)methanesulfonamide (Compound No.3) is described as Compound B6 in WO 2011/116951.

1-[3-({4-[2-(Benzyloxy)phenyl]-1,3,5-triazin-2-yl}amino)phenyl]methanesulfonamide (Compound No.4b) and trifluoroacetate thereof (Compound No.4a) are described as Compound B13 in WO 2011/116951.

4-{2-[(3,4-Dichlorophenyl)methoxy]phenyl}-N-{3-[(S-methanesulfonimidoyl)methyl]phenyl}-1,3,5-triazin-2-amine (Compound No.5) is described in Example 61 of WO 2012/160034.

Ethyl {[(3-{[4-(2,3-dihydro-1,4-benzodioxin-5-yl)-1,3,5-triazin-2-yl]amino}phenyl)methyl](methyl)oxo-$\lambda^6$-sulfanylidene}carbamate (Compound No.6) is described in Example 37 of WO 2012/160034.

Ethyl {[(3-{[4-(2,3-dihydro-1-benzofuran-7-yl)-1,3,5-triazin-2-yl]amino}phenyl)methyl] (methyl)oxo-$\lambda^6$-sulfanylidene}carbamate (Compound No.7) is described in Example 33 of WO 2012/160034.

Experimental Example 1

Using ALS7 (C9orf72) cell line described in K. Imamura et al, Science Translational Medicine 2017, 9, eaaf3962, the effect of the compound used in the present invention on RNA foci was examined. The cell line is stable cell line prepared by introducing the tetracycline-induced Lhx3, Ngn2 and Isl1 genes into iPS cell lines established from a familial ALS patient with abnormal expansion of the G4C2 repeat of the C9orf72 gene. Therefore, ALS7 (C9orf72) cell is an ALS cell model that rapidly (within about 7 days) differentiates into motor neurons when tetracycline or a derivative thereof is added to the medium, and after differentiation, spontaneously goes on to cell death (see the above documents). Moreover, the present inventors have found that the generation of RNA foci and DPRs occurs spontaneously prior to the cell death.

ALS7 (C9orf72) cells were cultured on feeder cells (mitomycin-treated SNL cells) using an iPS cell maintenance

medium consisting of Primate ES Cell medium (ReproCell, RCHEMD001A), 4 ng/ml hbFGF (Wako, 060-04543), 50 ug/ml G418 (Nacalai, 09380-86) and Penicillin-Streptomycin (Thermo Fisher Scientific, 15140-122).

The method for seeding ALS7 (C9orf72) cells on an assay plate is as follows.

Matrigel (BD Falcon, D2650) was diluted 20-fold with an assay medium consisting of DMEM/F-12 (1:1) (Thermo Fisher Scientific, 11330-057), N2 supplement (Thermo Fisher Scientific, 17502-048), Penicillin-Streptomycin (Thermo Fisher Scientific, 15140-122), 10 ng/ml recombinant human BDNF (PeproTech, 450-02), 10 ng/ml recombinant human GDNF (PeproTech, 450-10), 10 ng/ml recombinant human NT-3 (PeproTech, 450-03), 1 μM Retinoic acid (Sigma, R2625), 1 ug/ml Doxycycline (Clontech, 631311), 1 μM SAG (Enzo life sciences, ALX-270-426-M001) and 10 μM Y-27632 (Wako, 253-00513), and then a 384-well plate (CellCarrier-384 Ultra, PerkinElmer, 6057300) was coated with the diluted solution.

The ALS7 (C9orf72) cells were then suspended in the assay medium, and seeded on the matrigel-coated assay plate at $1\times10^4$ cells per well.

The fluorescent staining method of RNA foci in motor neurons differentiated from ALS7 (C9orf72) cells is as follows.

For the ALS7 (C9orf72) cells seeded on the assay plate according to the method described in the previous section, the assay medium containing no Y-27632 was added to the plate four days after seeding, and the cells were cultured. Seven days after seeding, the assay medium (Retinoic Acid, Doxycycline, SAG, Y-27632-free) containing the test compound at a predetermined concentration was added to the plate by medium exchange, and one day after addition of the test compound, PFA (Wako, 163-20145) was added to the plate to fix the cells. After washing with PBS (Wako, 045-29795) was repeated three times, ice-cold methanol (Wako, 131-01826) was added to the plate, and the plate was allowed to stand for 10 minutes at room temperature. DNA probe [5'-(Cy3)-CCCCGGCCCCGGCCCCGGCCCCGG-3' (SEQ ID NO:1), SIGMA genosys, custom synthesis] was denatured at 80°C for 75 sec, prepared to 2 μg/μL in a hybri buffer consisting of 50% formamide (Wako, 066-02301), 2xSSC (Nippon Gene, 319-90015), 50 mM sodium phosphate (TEKNOVA, P2070), 10% Dextran sulfate (SIGMA, D8906-100G), 0.1 mg/mL yeast tRNA (INVITRON, 15401029) and RNase free water (QIAGEN, 129112), and added to the plate, and the plate was allowed to stand at 37°C for 16 to 24 hrs to bind the probe to the RNA foci. A wash buffer consisting of 50% formamide, 1xSSC and RNase free water was added to the plate, and the plate was allowed to stand at 37°C for 30 minutes, followed by washing with PBS. Hoechst (invitrogen, H3569) diluted 5000-fold with by PBS was added to the plate, and the plate was allowed to stand for 20 minutes at room temperature to stain the cell nuclei, followed by washing with PBS.

The RNA foci in the cell nuclei were detected by analyzing the above treated plate (measuring Cy3 fluorescence) with a high-content analyzer. The high-content analyzer used was Opera Phenix from PerkinElmer. Figure 1 shows a typical image acquired by Opera.

The method for detecting the activity of the test compound is as follows.

As a negative control, the cells (motor neurons induced to differentiate from ALS7 (C9orf72)) cultured in the assay medium supplemented with DMSO instead of the test compound were used. Since in some cases the cells were layered and the cell nuclei overlapped with each other, the nuclear staining area was used as an index of the number of cells. The change in the number of cells was corrected by dividing the number of the RNA foci in the nuclei by the nuclear staining area.

The degree to which the test compound reduced the number of the RNA foci in the negative control is defined as the RNA foci inhibitory activity of the test compound, which was calculated by the following formula.

$$\text{RNA foci inhibitory activity of the test compound} = 100 - X/C \times 100$$

X: Number of RNA foci per constant nuclear area in the test compound group,
C: Number of RNA foci per constant nuclear area in the DMSO group

The activity at the test compound concentrations of 1, 3 and 10 μmol/l are shown in the following Table 2.

[Table 2]

| Compound No. | RNA foci inhibitory activity % | | |
|---|---|---|---|
| | 1 μM | 3 μM | 10 μM |
| 1 | 48 | 58 | 46 |
| 2 | 20 | 49 | 55 |

(continued)

| Compound No. | RNA foci inhibitory activity % | | |
|---|---|---|---|
| | 1 μM | 3 μM | 10 μM |
| 3 | 16 | 55 | 51 |
| 4a | 50 | 45 | 45 |
| 4b | 52 | 41 | 47 |
| 5 | 61 | 44 | 50 |
| 6 | 62 | 48 | 48 |
| 7 | 47 | 55 | 43 |

As shown in Table 2, when treated with any of the compounds, the spontaneous RNA foci generation in ALS7 (C9orf72) cell-derived motor neurons was effectively inhibited. In particular, Compound Nos. 1, 4a, 4b and 5-7 inhibited the RNA foci generation by 40% or more over a wide concentration range of 1-10 μM.

Therefore, it was shown that the compound according to the present invention can effectively inhibit the RNA foci generation caused by abnormal expansion of the G4C2 repeat.

Experimental Example 2

Next, the effect of the compound used in the present invention on RAN translation was examined. Among the DPRs generated by RAN translation, the poly-GP level was measured by an electrochemiluminescence system (Meso Scale Diagnostics).

ALS7 (C9orf72) cells were seeded on an assay plate in the same procedure as in Experimental Example 1. Four days after seeding, the assay medium containing no Y-27632 was added to the plate, and the cells were cultured until seven days after seeding, and the assay medium (Retinoic Acid, Doxycycline, SAG, Y-27632-free) containing the test compound at a predetermined concentration was added to the plate by medium exchange. Two days after addition of the test compound, the medium was removed, and the cells were lysed in an urea buffer consisting of 8M Urea (Wako, 219-00175), 4% CHAPS (Dojindo, 349-04722) and 30 mM Tris-HCl (pH 8.0, Nippon Gene, 312-90061). The cell lysate was added to a multi-array 384 well plate (Meso Scale Diagnostics, L21XB-4), and the plate was agitated at 700 rpm with a plate shaker (Azwan, DM-301) at room temperature, and then allowed to stand overnight at 4°C. The cell lysate was removed, a blocking buffer consisting of 5% Blocker A (Meso Scale Diagnostics, R93AA-1) and 1xTBS-T {TBS (Bio-Rad, 170-6435) containing 0.05% Tween-20 (Bio-Rad, 170-6531)} was added to the plate, and the plate was agitated at 700 rpm with a plate shaker for 1 hr at room temperature, and then washed three times with a wash buffer consisting of 0.05% Tween-20 and PBS (Wako, 162-18547). C9RANT antibody (Novus Biologicals, NBP2-25018) diluted 10,000-fold with 1xTBS-T containing 1% Blocker A was added to the plate, and the plate was agitated at 700 rpm with a plate shaker for 2 hr at 4°C, and then allowed to stand overnight at 4°C. After washing three times with the wash buffer, SULFO-TAG Goat Anti-Rabbit Antibody (Meso Scale Diagnostics, R32AB-1) diluted 500-fold with 1xTBS-T containing 1% Blocker A was added to the plate, and the plate was agitated at 700 rpm with a plate shaker for 2 hr at room temperature. After washing three times with the wash buffer, 2xMSD Read Buffer T with Surfactant (Meso Scale Diagnostics, R92TC-1) was added to the plate, and the signal derived from the C9RANT antibody was measured by MESO SECTOR S600 (Meso Scale Diagnostics).

The method for detecting the activity of the test compound is as follows.

As a negative control, the cells cultured in the assay medium supplemented with DMSO instead of the test compound were used.

The degree to which the test compound reduced the poly-GP level in the negative control is defined as the RAN translation inhibitory activity of the test compound, which was calculated by the following formula.

$$\text{RAN translation inhibitory activity of the test compound} = 100 - X/C \times 100$$

X: poly-GP level in the test compound group,
C: poly-GP level in the DMSO group

The activity at the test compound concentrations of 1, 3 and 10 μmol/l are shown in the following Table 3.

[Table 3]

| Compound No. | RAN translation inhibitory activity % | | |
|---|---|---|---|
| | 1 μM | 3 μM | 10 μM |
| 1 | 1 | 19 | 18 |
| 2 | > 1 | 6 | 15 |
| 3 | > 1 | 1 | 4 |
| 4a | 5 | 11 | 3 |
| 4b | 11 | 17 | 17 |
| 5 | 19 | 16 | 21 |
| 6 | 16 | - | 21 |
| 7 | 4 | 19 | 17 |

As shown in Table 3, when treated with any of the compounds, the poly-GP generation in ALS7 (C9orf72) cell-derived motor neurons was effectively inhibited.
Therefore, it was shown that the compound according to the present invention can effectively inhibit the RAN translation caused by abnormal expansion of the G4C2 repeat.

Experimental Example 3

In order to examine the cytotoxicity of the test compound, the intracellular ATP level was measured.
ALS7 (C9orf72) cells were seeded on an assay plate in the same procedure as in Experimental Example 1. Four days after seeding, the assay medium containing no Y-27632 was added to the plate, and the cells were cultured until seven days after seeding, and the assay medium (Retinoic Acid, Doxycycline, SAG, Y-27632-free) containing the test compound at a predetermined concentration was added to the plate by medium exchange. 24 hr or 48 hr after addition of the test compound, the medium was removed, and CellTiter-Glo Luminescent Cell Viability Assay (Promega, G7570) was performed.
The method for detecting the cytotoxicity of the test compound is as follows.
As a negative control, the cells cultured in the assay medium supplemented with DMSO instead of the test compound were used.
The degree of the effect on the intracellular ATP level in the test compound-treated cells was defined as the ATP level of the test compound compared with the negative control, which was calculated by the following formula.

$$\text{ATP level of the test compound compared to the negative control} = X/C \times 100$$

X: ATP level in the test compound group,
C: ATP level in the DMSO group

When the intracellular ATP level was less than 0.5 times or more than 1.5 times the intracellular ATP level in the negative control, it was judged to be cytotoxic. The intracellular ATP levels at the test compound concentrations of 1, 3 and 10 μmol/l are shown in the following Table 4.

[Table 4]

| Compound No. | ATP level % compared to negative control | | | | | |
|---|---|---|---|---|---|---|
| | After 24 hr | | | After 48 hr | | |
| | 1 μM | 3 μM | 10 μM | 1 μM | 3 μM | 10 μM |
| 1 | 92 | 107 | 134 | 91 | 89 | 122 |

(continued)

| Compound No. | ATP level % compared to negative control | | | | | |
|---|---|---|---|---|---|---|
| | After 24 hr | | | After 48 hr | | |
| | 1 μM | 3 μM | 10 μM | 1 μM | 3 μM | 10 μM |
| 2 | 98 | 89 | 101 | 99 | 74 | 53 |
| 3 | 104 | 97 | 106 | 105 | 87 | 69 |
| 4a | 113 | 128 | 140 | 80 | 101 | 115 |
| 4b | 105 | 121 | 134 | 73 | 97 | 102 |
| 5 | 98 | 118 | 131 | 83 | 94 | 108 |
| 6 | 89 | 107 | 130 | 77 | 71 | 124 |
| 7 | 89 | 105 | 126 | 86 | 87 | 112 |

As shown in Table 4, when treated with any of the compounds, the intracellular ATP levels in the ALS7 (C9orf72) cell-derived motor neurons ranges within from 0.5 to 1.5 times the intracellular ATP levels in the negative control. Even when each compound was treated for 48 hours, the levels were within the above range. Therefore, the cytotoxicity of the compound according to the present invention is considered to be sufficiently low. In particular, for compounds 1, 6 and 7, the intracellular ATP levels were maintained at 80% or more of the negative control even when treated in the concentration range of 1-10 μM for 48 hours. Therefore, the cytotoxicity is considered to be extremely low.

As is clear from these results, it was shown that the compound according to the present invention can effectively inhibit the RNA foci and RAN translation caused by abnormal expansion of the G4C2 repeat, within a concentration range with sufficiently low cytotoxicity.

The compounds of Reference Examples 1 to 30 shown in the following Table 5 can also be expected to inhibit the RNA foci and RAN translation, as well as Compound Nos. 1 to 7.

[Table 5-1]

| Ref. Ex. No. | Structure |
|---|---|
| Ref. Ex. 1 | 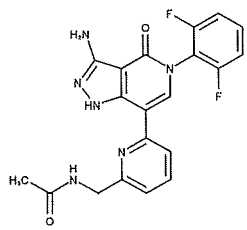 |
| Ref. Ex. 2 | 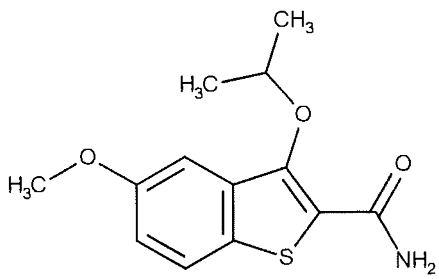 |
| Ref. Ex. 3 | 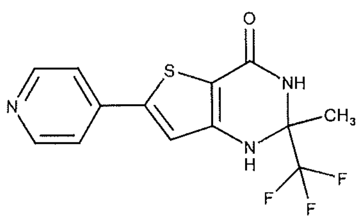 |

(continued)

| Ref. Ex. No. | Structure |
|---|---|
| Ref. Ex. 4 | 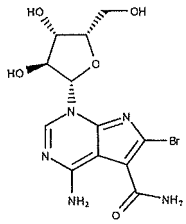 |
| Ref. Ex. 5 | 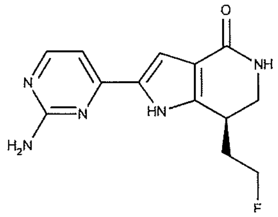 |
| Ref. Ex. 6 | 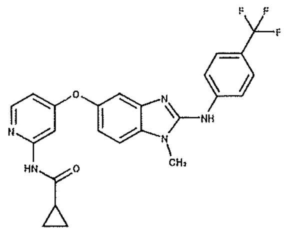 |

[Table 5-2]

| | |
|---|---|
| Ref. Ex. 7 | 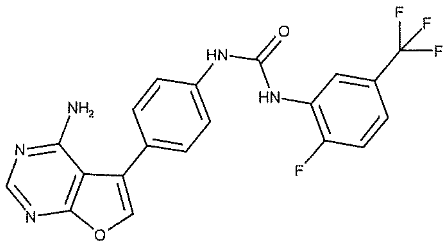 |
| Ref. Ex. 8 | 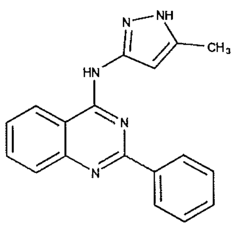 |
| Ref. Ex. 9 | 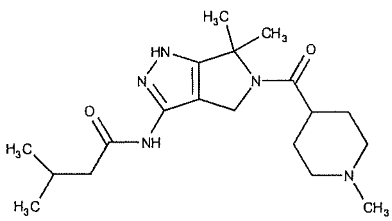 |

(continued)

| | |
|---|---|
| Ref. Ex. 10 | 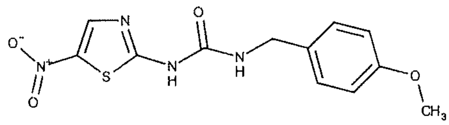 |
| Ref. Ex. 11 | 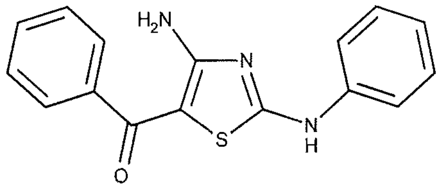 |
| Ref. Ex. 12 | 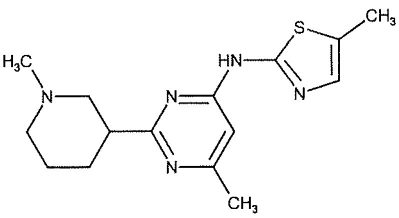 |

[Table 5-3]

| | |
|---|---|
| Ref. Ex. 13 | 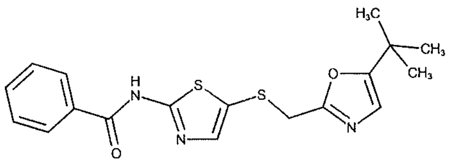 |
| Ref. Ex. 14 | 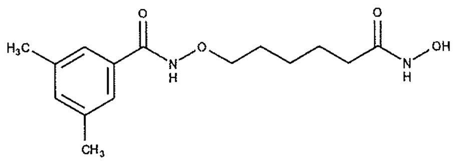 |
| Ref. Ex. 15 | 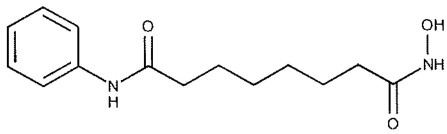 |
| Ref. Ex. 16 | 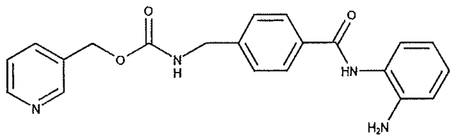 |
| Ref. Ex. 17 | 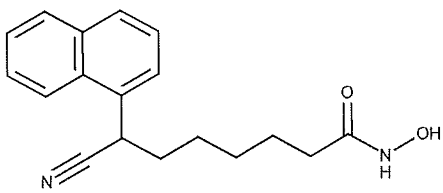 |

(continued)

| | |
|---|---|
| Ref. Ex. 18 | 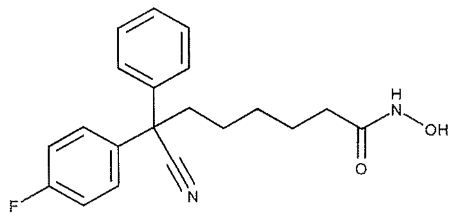 |

[Table 5-4]

| | |
|---|---|
| Ref. Ex. 19 | 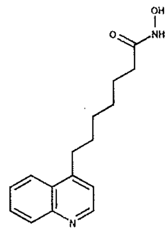 |
| Ref. Ex. 20 | 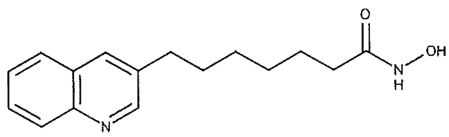 |
| Ref. Ex. 21 | 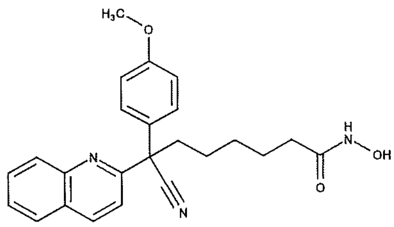 |
| Ref. Ex. 22 | 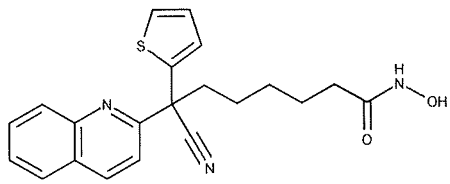 |
| Ref. Ex. 23 | 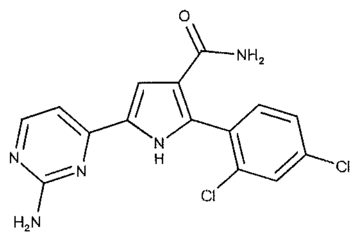 |

(continued)

| | |
|---|---|
| Ref. Ex. 24 | 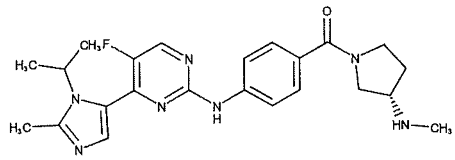 |

[Table 5-5]

| | |
|---|---|
| Ref. Ex. 25 | 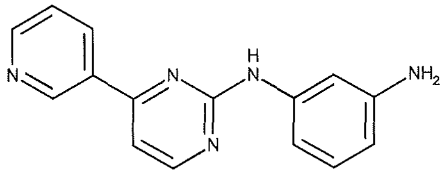 |
| Ref. Ex. 26 | 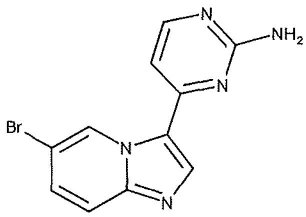 |
| Ref. Ex. 27 | 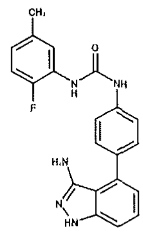 |
| Ref. Ex. 28 | 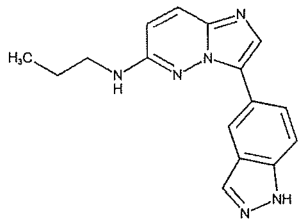 |
| Ref. Ex. 29 | 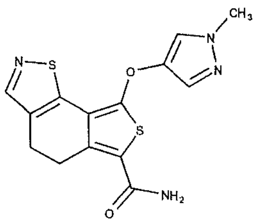 |
| Ref. Ex. 30 | 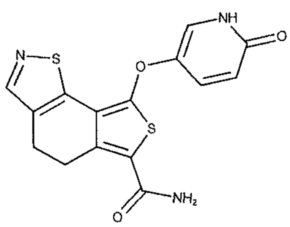 |

Formulation Examples

Medicaments containing the compound of the present invention as an active ingredient can be produced, for example, by the following formulations.

1. capsule

| | |
|---|---|
| (1) compound obtained in Example 1 | 10 mg |
| (2) lactose | 90 mg |
| (3) microcrystalline cellulose | 70 mg |
| (4) magnesium stearate | 10 mg |
| 1 capsule | 180 mg |

The total amount of the above-mentioned (1), (2) and (3) and 5 mg of (4) are blended and granulated, and 5 mg of the remaining (4) is added. The whole mixture is sealed in a gelatin capsule.

2. tablet

| | |
|---|---|
| (1) compound obtained in Example 1 | 10 mg |
| (2) lactose | 35 mg |
| (3) cornstarch | 150 mg |
| (4) microcrystalline cellulose | 30 mg |
| (5) magnesium stearate | 5 mg |
| 1 tablet | 230 mg |

The total amount of the above-mentioned (1), (2) and (3), 20 mg of (4) and 2.5 mg of (5) are blended and granulated, and 10 mg of the remaining (4) and 2.5 mg of the remaining (5) are added and the mixture is compression formed to give a tablet.

**Industrial Applicability**

The compound according to the present invention can effectively inhibit the generation of RNA foci and the generation of DPRs by RAN translation in neurodegenerative diseases (for example, ALS or FTD) caused by abnormal expansion of the G4C2 repeat, and can prevent or treat such diseases. Some neurodegenerative diseases result from abnormal expansion of repeat consisting of different hexa- (or tri-) nucleotide sequences, and in any case, the generation of RNA foci and DPRs is considered to be the main cytotoxicity leading to neurodegeneration. And, it has been suggested that there is a common mechanism that is sequence-independent in the process of the generation of RNA foci and DPRs.
Therefore, the compound according to the present invention is expected to contribute as an agent for the prophylaxis or treatment of not only neurodegenerative diseases caused by abnormal expansion of the G4C2 repeat but also all neurodegenerative diseases caused by abnormal expansion of nucleotide repeat.
This application is based on patent applications No. 2020-058414 filed on March 27, 2020 in Japan, the contents of which are encompassed in full herein.

**Claims**

**1.** A neuron degeneration inhibitor comprising a compound represented by the formula (I)

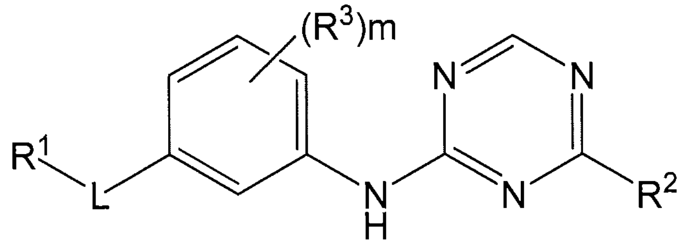

(I)

wherein

$R^1$ is a group represented by the formula (a-1) or (a-2)

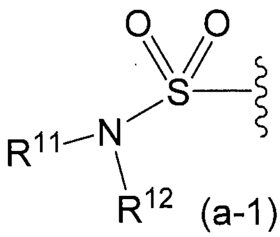

(a-1) or

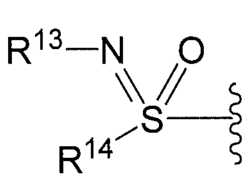

(a-2)

wherein

$R^{11}$ and $R^{12}$ are each independently a hydrogen atom or a $C_{1-6}$ alkyl group,
$R^{13}$ is a hydrogen atom, a cyano group, a $C_{1-6}$ alkyl-carbonyl group or a $C_{1-6}$ alkoxy-carbonyl group, and
$R^{14}$ is a $C_{1-6}$ alkyl group, a $C_{3-8}$ cycloalkyl group or a $C_{6-14}$ aryl group,

$R^2$ is a group represented by the formula (b-1)-(b-3)

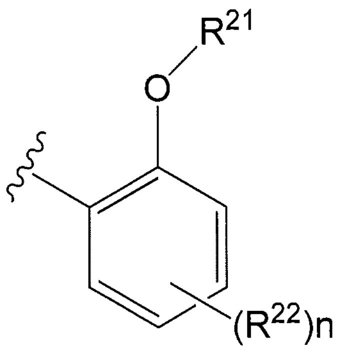

(b-1)

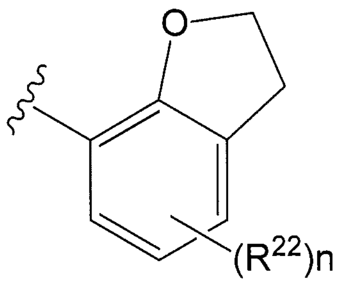

(b-2) or

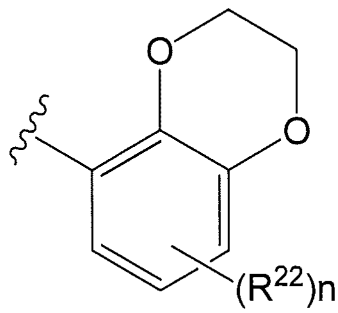

(b-3)

wherein

$R^{21}$ is a $C_{1-6}$ alkyl group, a $C_{6-14}$ aryl group optionally substituted by halogen atom(s), or a $C_{7-16}$ aralkyl group optionally substituted by halogen atom(s),
$R^{22}$ is each independently a halogen atom, a cyano group, a $C_{1-6}$ alkyl group or a $C_{1-6}$ alkoxy group, and
n is 0, 1 or 2,

$R^3$ is each independently a halogen atom, a cyano group, a $C_{1-6}$ alkyl group or a $C_{1-6}$ alkoxy group,
m is 0, 1 or 2, and
L is a $C_{1-3}$ alkylene group,
or a salt thereof.

**2.** The neuron degeneration inhibitor according to claim 1, wherein

$R^{11}$ and $R^{12}$ are both hydrogen atoms,
$R^{13}$ is a hydrogen atom or a $C_{1-6}$ alkoxy-carbonyl group,
$R^{14}$ is a $C_{1-6}$ alkyl group,
$R^{21}$ is a $C_{1-6}$ alkyl group, or a $C_{7-16}$ aralkyl group optionally substituted by halogen atom(s),
$R^{22}$ is a halogen atom,
n is 0 or 1,
m is 0, and
L is a methylene group.

**3.** The neuron degeneration inhibitor according to claim 1, wherein the compound represented by the formula (I) or a salt thereof is selected from

(1) 4-(4-fluoro-2-methoxyphenyl)-N-{3-[(S-methanesulfonimidoyl)methyl]phenyl}-1,3,5-triazin-2-amine,
(2) 1-(3-{[4-(2-methoxyphenyl)-1,3,5-triazin-2-yl]amino}phenyl)methanesulfonamide,
(3) 1-(3-{[4-(4-chloro-2-methoxyphenyl)-1,3,5-triazin-2-yl]amino}phenyl)methanesulfonamide,
(4) 1-[3-({4-[2-(benzyloxy)phenyl]-1,3,5-triazin-2-yl}amino)phenyl]methanesulfonamide,
(5) 4-{2-[(3,4-dichlorophenyl)methoxy]phenyl}-N-{3-[(S-methanesulfonimidoyl)methyl]phenyl}-1,3,5-triazin-2-amine,
(6) ethyl {[(3-{[4-(2,3-dihydro-1,4-benzodioxin-5-yl)-1,3,5-triazin-2-yl]amino}phenyl)methyl] (methyl)oxo-$\lambda^6$-sulfanylidene}carbamate,
(7) ethyl {[(3-{[4-(2,3-dihydro-1-benzofuran-7-yl)-1,3,5-triazin-2-yl]amino}phenyl)methyl] (methyl)oxo-$\lambda^6$-sulfanylidene}carbamate,

and salts thereof.

**4.** The neuron degeneration inhibitor according to any of claims 1 to 3, which is used as an agent for the prophylaxis or treatment of a motor neuron disease or dementia.

**5.** The neuron degeneration inhibitor according to claim 4, wherein the motor neuron disease or dementia is a motor neuron disease or dementia involving abnormal expansion of hexanucleotide repeat.

**6.** The neuron degeneration inhibitor according to claim 4 or 5, wherein the motor neuron disease is amyotrophic lateral sclerosis.

**7.** The neuron degeneration inhibitor according to claim 4 or 5, wherein the dementia is frontotemporal dementia.

**8.** A method for inhibiting neuron degeneration in a mammal, which comprises administering an effective amount of a compound or salt as defined in any of claims 1 to 3 to the mammal.

**9.** A method for preventing or treating a neuron degeneration disease in a mammal, which comprises administering an effective amount of a compound or salt as defined in any of claims 1 to 3 to the mammal.

**10.** A compound or salt as defined in any of claims 1 to 3 for use in the prophylaxis or treatment of a neuron degeneration disease.

**11.** Use of a compound or salt as defined in any of claims 1 to 3 for the manufacture of an agent for the prophylaxis or treatment of a neuron degeneration disease.

Fig. 1

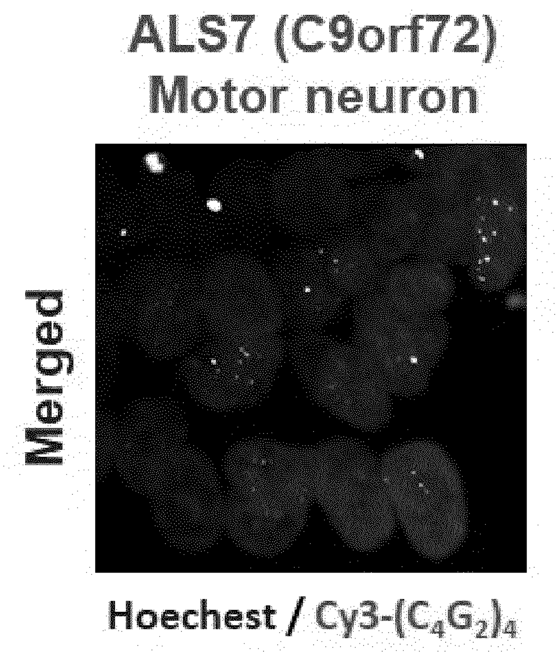

## INTERNATIONAL SEARCH REPORT

International application No.
PCT/JP2021/014251

A. CLASSIFICATION OF SUBJECT MATTER
A61K 31/53(2006.01)i; A61P 21/02(2006.01)i; A61P 25/00(2006.01)i; A61P 25/28(2006.01)i; C07D 251/22(2006.01)i; C07D 405/04(2006.01)i
FI: A61K31/53; A61P21/02; A61P25/00; A61P25/28; C07D251/22 C; C07D405/04
According to International Patent Classification (IPC) or to both national classification and IPC

B. FIELDS SEARCHED
Minimum documentation searched (classification system followed by classification symbols)
A61K31/53; A61P21/02; A61P25/00; A61P25/28; C07D251/22; C07D405/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2014-515363 A (BAYER INTELLECTUAL PROPERTY GMBH) 30 June 2014 (2014-06-30) paragraphs [0899]-[0911] | 10<br>1-9, 11 |
| X<br>A | WO 2013/026874 A1 (LEAD DISCOVERY CENTER GMBH) 28 February 2013 (2013-02-28) pp. 31-37, table 1 | 10<br>1-9, 11 |
| X<br>A | WO 2019/217757 A1 (DESIGN THERAPEUTICS INC.) 14 November 2019 (2019-11-14) p. 156 | 10<br>1-9, 11 |
| A | WO 2019/067587 A1 (UNIVERSITY OF FLORIDA RESEARCH FOUNDATION, INCORPORATED) 04 April 2019 (2019-04-04) claims | 1-11 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 May 2021 (12.05.2021) | 25 May 2021 (25.05.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/014251

| Patent Documents referred in the Report | Publication Date | Patent Family | Publication Date |
|---|---|---|---|
| JP 2014-515363 A | 30 Jun. 2014 | WO 2012/160034 A1<br>pp. 180-191<br>EP 2527332 A1<br>US 2014/0315906 A1<br>CN 103702979 A<br>KR 10-2014-0041571 A | |
| WO 2013/026874 A1 | 28 Feb. 2013 | EP 2561867 A1<br>US 2014/0303167 A1 | |
| WO 2019/217757 A1 | 14 Nov. 2019 | EP 3790872 A1 | |
| WO 2019/067587 A1 | 04 Apr. 2019 | JP 2020-516674 A<br>US 2020/0140846 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018193309 A
- WO 2014148646 A
- WO 2012160034 A
- WO 2011116951 A
- WO 2020058414 A

### Non-patent literature cited in the description


- **DEJESUS-HERNANDEZ, M. et al.** *Neuron,* 2011, vol. 72, 245-56
- **RENTON, A.E. et al.** *Neuron,* 2011, vol. 72, 257-68
- **ASH, P.E. et al.** *Neuron,* 2013, vol. 77, 639-46
- **MORI, K. et al.** *Science,* 2013, vol. 339, 1335-8
- **ZU, T. et al.** *Proc Natl Acad Sci U S A,* 2013, vol. 110, E4968-77
- **J. CHEW et al.** *Science,* 2015, vol. 348, 1151-1154
- **D. SAREEN et al.** *Sci. Transl. Med.,* 2013, vol. 5, 208ra149
- **K. IMAMURA et al.** *Science Translational Medicine,* 2017, vol. 9, eaaf3962